# EUROPÄISCHE PATENTSCHRIFT

(11) **EP 2 533 908 B1**
(45) Veröffentlichungstag und Bekanntmachung des Hinweises auf die Patenterteilung: **12.08.2015**
(21) Anmeldenummer: 10787262.4
(22) Anmeldetag: 25.11.2010
(51) Int. Cl.: B05C 17/01, A61M 5/315, B05C 17/005, A61M 5/19

(54) **AUSTRAGVORRICHTUNG MIT RASTELEMENT**
DISCHARGE DEVICE HAVING A LOCKING ELEMENT
DISPOSITIF DE DISTRIBUTION PRÉSENTANT UN ÉLÉMENT D'ENCLIQUETAGE

(30) Priorität: 12.02.2010 CH 17212010
(43) Veröffentlichungstag der Anmeldung: 19.12.2012
(73) Patentinhaber: Medmix Systems AG, 6343 Rotkreuz (CH)
(72) Erfinder: KAYSER, Ralph, Egon, CH-6004 Luzern (CH); BREM, William, CH-5630 Muri (CH)
(74) Vertreter: Detken, Andreas
(86) Internationale Anmeldenummer: PCT/CH2010/000300
(87) Internationale Veröffentlichungsnummer: WO 2011/097742

(56) Entgegenhaltungen:
- WO-A1-2009/125353
- WO-A2-2008/057976
- DE-A1- 3 701 500
- DE-A1- 3 925 681

## Beschreibung

### TECHNISCHES GEBIET

Die vorliegende Erfindung betrifft eine Austragvorrichtung zum Austragen eines fluiden Produktes mit einem Gehäuse und einem darin verschiebbaren Kolben.

### STAND DER TECHNIK

Austragvorrichtungen in Form von Spritzen sind seit langem allgemein bekannt. Eine handelsübliche Spritze weist einen Spritzenkörper mit einer distalen Austrittsöffnung und eine Kolbeneinheit auf, die von einem proximalen Ende her in den Spritzenkörper einschiebbar ist. Die Kolbeneinheit weist eine Kolbenstange mit einem distal daran angebrachtem Kolben auf, der dichtend im Innenraum des Spritzenkörpers verschiebbar ist. Um ein Fluid in der Spritze aufzunehmen, kann die Kolbeneinheit in proximaler Richtung zurückgezogen werden, wodurch im Spritzenkörper ein Unterdruck entsteht und das Fluid durch die distale Austrittsöffnung eingesaugt wird. Beim anschliessenden Vorschieben der Kolbeneinheit in distaler Richtung wird das Fluid durch die Austrittsöffnung wieder ausgestossen.

Dabei besteht die Gefahr, dass die Kolbeneinheit unbeabsichtigt zu weit aus dem Spritzenkörper zurückgezogen wird und dabei aus dem diesem herausfällt, so dass das in der Spritze aufgenommene Fluid verschüttet wird und die Umgebung kontaminiert. Häufig ist es zudem erwünscht, nur eine bestimmte Menge ("Dosis") des Fluids aus der Spritze auszutragen. Eine solche Dosierung oder Portionierung ist mit handelsüblichen Spritzen nur ungenau durch Ablesen einer aufgedruckten Skala möglich. Dabei besteht immer die Gefahr, dass unbeabsichtigt zu viel Fluid abgegeben wird. Beim Aufnehmen des Fluids besteht umgekehrt das Problem, dass aufgrund des Unterdrucks, welcher im Spritzenkörper vorhanden ist, der Kolben beim Loslassen wieder ein wenig in Richtung der. Austrittsöffnung zurückschnellt, wodurch die endgültig aufgezogene Menge des Fluids kleiner als erwünscht ist.

CA 2 036 940, WO 2009/144085, US 2,856,925, und US 4,874,385 beschreiben jeweils eine Spritze, bei der in einem ausserhalb des Spritzengehäuses liegenden Bereiches der Kolbenstange ein Stoppelement angebracht ist. Beim Vorschieben der Kolbeneinheit schlägt das Stoppelement am proximalen Gehäuseende an und verhindert so ein weiteres Vorschieben. Durch die entsprechende Anordnung des Stoppelements an der Kolbenstange kann die zu verabreichende Dosis eingestellt werden. Die Bewegung der Kolbeneinheit in die proximale Richtung ist bei diesen Spritzen jedoch nicht limitiert.

US 2,373,520, US 2,875,761 und US 2,474,496 offenbaren Spritzen, an deren proximalem Gehäuseende aussen ein Federelement angebracht ist. Dieses Federelement ist dazu ausgebildet, ausserhalb des Spritzengehäuses in Rastkerben an der Kolbenstange einzurasten, um dadurch das erfolgte Austragen einer bestimmten Dosis anzuzeigen. US 2009/0308891 offenbar verschiedene Ausführungsformen einer Doppelspritze mit Doppelkolben. In einer Ausführungsform sind am proximalen Ende der Doppelspritze Rastnasen vorhanden, die in Rastkerben der beiden Kolbenstangen des Doppelkolbens eingreifen. In einer anderen Ausführungsform ist mit dem Doppelkolben eine separate Raststange verbunden, die parallel zu den Kolbenstangen verläuft und an der Rastkerben ausgebildet sind. Wiederum sind am proximalen Ende der Doppelspritze Rastnasen vorhanden, die in diese Rastkerben eingreifen. Ein versehentliches Entfernen der Kolbeneinheit vom Gehäuse ist jedoch auch bei allen vorgenannten Spritzen möglich.

Eine weitere derartige Spritze mit Federelement, bei der aber ein komplettes Entfernen der Kolbeneinheit vom Gehäuse verhindert wird, ist in US 2,707,954 offenbart. Die Bauweise dieser Spritze ist jedoch insgesamt aufwändig und komplex. Die Spritze ist zudem umständlich in der Handhabung.

US 4,386,606 und US 4,711,637 offenbaren Spritzen, bei denen die Kolbeneinheit am Gehäuse mittels eines Festklemmelementes blockierbar ist. Das Festklemmelement bildet ausserdem einen proximalen. Anschlag für den Kolben und verhindert dadurch ein vollständiges Herausziehen des Kolbens aus dem Gehäuse. Ein proximaler Kolbenanschlag, der dazu dient, ein Entfernen einer Kolbeneinheit von einem Gehäuse zu verhindern, ist auch in US 772,114 offenbart. Die in diesen Dokumenten gezeigten proximalen Anschlagelemente sind alle lösbar aussen am proximalen Ende des Gehäuses angeordnet. Es besteht dadurch die Gefahr, dass das Anschlagelement während der Hantierung mit der Austragvorrichtung verrutscht oder sich gar löst.

Die WO 2009/125353 beschreibt eine Austragvorrichtung mit einem Reservoir und einem an einer Kolbenstange angebrachten Kolben. In einer sich um den Kolben und die Kolbenstange herum erstreckenden Seitenwand ist eine Fensteröffnung ausgebildet, in welche ein Rastelement eingesetzt ist. Dieses Rastelement steht mit der gezackt ausgebildeten Kolbenstange in Eingriff und dient dazu, den Kolben in distaler Richtung zu verschieben.

Bei dem in DE 39 25 681 gezeigten Auftragsgerät ist eine gezackte Kolbenstange mittels eines Federelements, welches für den Benutzer durch eine seitliche Öffnung hindurch betätigbar ist, in Längsrichtung verschiebbar.

### DARSTELLUNG DER ERFINDUNG

Es ist eine Aufgabe der vorliegenden Erfindung, eine Austragvorrichtung anzugeben, welche so ausgebildet ist, dass sie ein vollständiges Entfernen des Kolbens aus dem Gehäuse verhindert und/oder dem Benutzer eine einfache Portionierung des ausgetragenen Produkts ermöglicht. Die Austragvorrichtung soll ausserdem eine sichere und einfache Handhabung sowie eine möglichst einfache Bauweise aufweisen.

Diese Aufgabe wird durch eine Vorrichtung mit den Merkmalen des Anspruchs 1 gelöst. Weitere Ausführungsformen sind in den abhängigen Ansprüchen angegeben.

Die vorliegende Erfindung stellt also eine Austragvorrichtung zum Austragen eines fluiden Produktes zu Verfügung, aufweisend
ein Gehäuse, welches ein Reservoir für das fluide Produkt begrenzt, mit einer umlaufenden Seitenwand, einem offenen proximalen Gehäuseende und einem distalen Gehäuseende mit einer Austrittsöffnung, und
einen Kolben, welcher im Gehäuse entlang einer Längsrichtung verschiebbar angeordnet ist, um das fluide Produkt durch die Austrittsöffnung hindurch aus dem Reservoir auszustossen,
wobei in der Seitenwand des Gehäuses eine Fensteröffnung ausgebildet ist, und
wobei die Austragvorrichtung ein Einsatzelement aufweist, welches quer zur Längsrichtung in die Fensteröffnung eingesetzt ist und dabei in das Reservoir hineinragt.

Das Einsatzelement erlaubt es, je nach konkreter Ausgestaltung ein vollständiges Entfernen des Kolbens vom Gehäuse zu verhindern und/oder dem Benutzer das Austragen einer bestimmten Menge des Fluids spürbar anzuzeigen.

Im Folgenden werden Richtungsangaben wie folgt verwendet. Die Längsrichtung bezeichnet die Verschiebungsrichtung des Kolbens. Die distale Richtung ist jeweils diejenige Richtung, in welche der Kolben zum Austragen eines in der Vorrichtung aufgenommenen Fluids zu einer Austrittsöffnung hin vorgeschoben wird. Die proximale Richtung bezeichnet die dazu entgegengesetzte Richtung.

Die Austragvorrichtung kann insbesondere als Spritze, mit einem dichtend im Gehäuse verschiebbaren Kolben ausgebildet sein. Das Gehäuse kann eine klassische zylindrische Spritzenform aufweisen, kann aber auch z.B. eine Kartusche bilden, deren äussere Gehäuseform von der klassischen Spritzenform abweicht. Der Kolben kann unmittelbar das Reservoir für das Fluid begrenzen, oder er kann indirekt auf das Reservoir einwirken, wobei das Reservoir beispielsweise durch einen im Gehäuse aufgenommenen Beutel oder einen faltenbalgähnlichen Behälter begrenzt ist. In einem solchen Fall braucht der Kolben nicht zwingend dichtend an der Seitenwand des Gehäuses anzuliegen, auch wenn dies in bevorzugten Ausführungsformen der Fall ist. Das distale Gehäuseende kann z.B. durch eine senkrecht oder schräg zur Längsrichtung verlaufende Wand gebildet sein oder auch eine andere Form annehmen. Die Austrittsöffnung weist bevorzugt in die distale Richtung entlang der Längsrichtung, kann aber auch gewinkelt zur Längsrichtung verlaufen.

Als Fluid wird eine beliebige fliessfähige Zustandsform bezeichnet. Der Begriff Fluid schliesst also niedrigviskose Flüssigkeiten, viskose Flüssigkeiten wie Öle oder Gele, Emulsionen, Suspensionen oder auch Pulver ein. Bei dem auszutragenden Fluid kann es sich insbesondere um ein Medikament, um eine Komponente eines zum mischenden Medikaments oder um eine medizinische oder nicht-medizinische Klebstoffkomponente handeln.

Die Fensteröffnung ist bevorzugt benachbart zum proximalen Gehäuseende angeordnet, insbesondere näher am proximalen als am distalen Gehäuseende. Sie ist vorzugsweise umlaufend von der Seitenwand umrandet und insbesondere vorzugsweise in proximaler Richtung vollständig durch die Seitenwand begrenzt. Sie kann aber auch in proximaler Richtung offen sein, solange sie in dieser Richtung durch ihre Formgebung einen Anschlag für das Einsatzelement bildet, der das Einsatzelement in proximaler Richtung fixiert. Vorteilhaft ist das Einsatzelement als Ganzes in einem Bereich der Seitenwand angeordnet, welcher sich beabstandet zum proximalen Gehäuseende befindet, befindet sich also vollständig distal beabstandet zum proximalen Gehäuseende, so dass das Einsatzelement nicht proximal über das proximale Gehäuseende hinausragt. Das Einsatzelement ist bevorzugt senkrecht zur Längsrichtung in die Fensteröffnung eingesetzt. Es schliesst nach aussen hin bevorzugt bündig mit dem Gehäuse ab, um ein unbeabsichtigtes Entfernen zu verhindern und eine einfache Handhabung der Vorrichtung sowie ein ansprechendes Erscheinungsbild zu ermöglichen.

Vorzugsweise weist das vom Gehäuse begrenzte Reservoir zumindest bereichsweise die Form eines Kreiszylinders auf, wobei die Zylinderachse die Längsrichtung definiert. Das Einsatzelement ist dann vorteilhaft in einer dazu senkrechten radialen Richtung von aussen her in die Fensteröffnung eingesetzt. Die Fensteröffnung erstreckt sich dann in Umfangsrichtung des Kreiszylinders über einen Winkelbereich von weniger als 180°, bevorzugt von weniger als 90°, insbesondere von 30-60°

Damit das Einsatzelement in der Fensteröffnung gehalten ist, kann es mindestens ein Hintergreifelement aufweisen. Das Hintergreifelement kann dabei insbesondere dazu dienen, das Einsatzelement in die Fensteröffnung einzuschnappen. Vorteilhaft schnappt das Hintergreifelement dabei auf einer Innenseite der Seitenwand ein. Das Einsatzelement kann zudem derart ausgestaltet sein, dass es nach erstmaligem Einschnappen in die Fensteröffnung nicht wieder aus dieser entfernbar ist. Dadurch kann beispielsweise aus hygienischen Gründen eine nur einmalige Verwendung der Austragvorrichtung garantiert werden. In anderen Ausführungsformen kann das Einsatzelement aber auch lösbar in die Fensteröffnung eingesetzt sein.

In einer bevorzugten Ausführungsform ist das Einsatzelement derart ausgebildet, dass es einen Anschlag für den Kolben bei einer Bewegung des Kolbens in Richtung der proximalen Gehäuseendes bildet. Dadurch wird ein unbeabsichtigtes Entfernen des Kolbens aus dem Gehäuse verhindert.

Erfindungsgemäß ist der Kolben mit einem Vorschubelement verbunden, das am proximalen Gehäuseende aus dem Gehäuse herausragt, z.B. in Form einer Kolbenstange. Der Kolben und das Vorschubelement können dabei voneinander lösbar oder fest miteinander verbunden sein und insbesondere einstückig ausgebildet sein. Das Vorschubelement weist erfindungsgemäß zumindest eine erste Raststruktur auf, und das Einsatzelement weist eine zweite Raststrukfur auf. Die Raststrukturen sind dazu ausgebildet, in mindestens einer Position des Vorschubelements entlang der Längsrichtung eine lösbare Rastverbindung einzugehen, um eine weitere Verschiebung des Vorschubelements in der Längsrichtung relativ zum Gehäuse zu erschweren. Auf diese Weise wird das Erreichen dieser Position beim Vorschieben des Vorschubelements dem Benutzer spürbar angezeigt, so dass der Benutzer das erfolgte Austragen einer bestimmten Portion des Fluids erkennt. Umgekehrt kann dem Benutzer beim Zurückziehen des Vorschubelementes dadurch eine bestimmte Menge des in die Austragvorrichtung aufgezogenen Fluids angezeigt werden. Ausserdem kann auf diese Weise verhindert werden, dass sich das Vorschubelement beim Zurückziehen in die proximale Richtung und anschliessendem Loslassen aufgrund einer Unterdruckbildung wieder ein Stück weit in die distale Richtung bewegt.

Die erste Raststruktur und die zweite Raststruktur sind erfindungsgemäß relativ zueinander in einer Eingriffsrichtung bewegbar, um miteinander die Rastverbindung einzugehen. Diese Eingriffsrichtung verläuft erfindungsgemäß sowohl quer zur Längsrichtung als auch quer zu derjenigen Richtung, in der das Einsatzelement in die Fensteröffnung eingesetzt ist. Auf diese Weise wirken Kräfte beim Eingreifen und Lösen der Rastverbindung derart, dass sie das Einsatzelement nicht aus der Fensteröffnung herauspressen können.

Vorteilhaft sind die erste Raststruktur und die zweite Raststruktur derart ausgebildet, dass die Rastverbindung durch Anwendung einer axialen Lösekraft entlang der Längsrichtung wieder lösbar ist. Insbesondere bevorzugt sind die erste Raststruktur und die zweite Raststruktur zudem derart ausgebildet, dass diese Lösekraft sowohl in die proximale als auch in die distale Richtung gerichtet sein kann. Dadurch lässt sich das Vorschubelement durch Überwindung eines erhöhten Druckwiderstands weiter in die distale Richtung vorschieben, um eine weitere Portion auszutragen, bzw. durch Überwindung einer erhöhten Zugkraft weiter zurückziehen, um eine weitere Portion aufzunehmen. Die axiale Lösekraft kann dabei in distaler Richtung unterschiedlich von der axialen Lösekraft in proximaler Richtung sein.

Das Vorschubelement kann insbesondere eine Mehrzahl von in axialer Richtung beabstandet zueinander angeordneten ersten Raststrukturen aufweisen. Die ersten Raststrukturen gehen dann mit der zweiten Raststruktur in unterschiedlichen Positionen des Vorschubelements eine lösbare Rastverbindung ein, indem sie bei einer axialen Bewegung des Vorschubelementes relativ zum Gehäuse nacheinander in die zweite Raststruktur des Einsatzelementes einrasten. Der Benutzer kann dadurch das Fluid in mehreren, wohldefinierten Portionen aus der Austragvorrichtung austragen. Dabei können die ersten Raststrukturen insbesondere in regelmässigen Abständen am Vorschubelement angeordnet sein.

Zumindest zwei der ersten Raststrukturen können vorteilhaft derart unterschiedlich zueinander ausgestaltet sein, dass die entlang der Längsrichtung wirkende axiale Lösekraft, die zum Lösen der Rastverbindung der jeweiligen ersten Raststruktur mit der zweiten Raststruktur erforderlich ist, jeweils unterschiedlich ist.

Die Raststrukturen können so ausgebildet sein, dass sie für mindestens eine Rastverbindung immer noch eine gewisse Bewegung des Vorschubelements entlang der Längsrichtung über einen bestimmten axialen Bewegungsbereich ("Spiel") erlauben, ohne dass dadurch die Rastverbindung gelöst wird. In diesem Fall ist es bevorzugt, dass dieser Bewegungsbereich im Bereich der distalen Anschlagstellung des Kolbens grösser als in anderen Bereichen ist. In anderen Worten ist der Kolben in eine distale Anschlagstellung verschiebbar, in der der Kolben in distaler Richtung am Gehäuse anschlägt, so dass eine weitere Bewegung des Kolbens und des Vorschubelementes relativ zum Gehäuse in distaler Richtung verhindert wird. Eine ausgewählte erste Raststruktur ist dann derart ausgebildet, dass sie eine Rastverbindung mit der zweiten Raststruktur eingeht, wenn sich der Kolben im Bereich seiner distalen Anschlagstellung befindet. Der axiale Bewegungsbereich des Vorschubelements für diese Rastverbindung ist dann grösser als für mindestens eine andere Rastverbindung einer der restlichen ersten Raststrukturen mit der zweiten Raststruktur. Dazu kann die entsprechende erste Raststruktur insbesondere länger bezüglich der Längsrichtung ausgebildet sein als mindestens eine andere der ersten Raststrukturen. Hierdurch wird es möglich, selbst nach Einrasten der Rastverbindung den Kolben bis zum distalen Anschlag zu drücken.

In einer konkreten Ausgestaltung kann die erste Raststruktur mindestens eine gegenüber der Längsrichtung geneigte Schrägfläche aufweisen, welche dazu ausgebildet ist, mit der zweiten Raststruktur zusammenzuwirken. Vorzugsweise weit die erste Raststruktur zwei entgegengesetzt geneigte Schrägflächen auf, die bei einer Bewegung des Vorschubelements in entgegengesetzte Richtungen jeweils mit der zweiten Raststruktur zusammenwirken. Die Neigungen dieser Schrägflächen können unterschiedlich sein, um unterschiedliche axiale Lösekräfte in distaler und proximaler Richtung zu erzielen.

Insbesondere kann die erste Raststruktur eine Rastkerbe im Vorschubelement umfassen, und die zweite Raststruktur weist dann zumindest eine Rastnase auf, die in der Raststellung in die Rastkerbe eingreift. Bevorzugt weist die erste Raststruktur zwei an gegenüberliegenden Seiten des Vorschubelements angeordnete Rastkerben auf, und die zweite Raststruktur weist entsprechend zwei von gegenüberliegenden Seiten in die Rastkerben eingreifende Rastnasen auf.

Insbesondere kann die zweite Raststruktur zwei flexibel ausgebildete Arme umfassen, die quer zur Längsrichtung einander gegenüberliegend angeordnet sind und jeweils eine zum anderen Arm hin gerichtete Rastnase aufweisen. Die erste Raststruktur ist dann derart ausgebildet, dass diese zwei Rastnasen von zwei gegenüberliegenden Seiten her in der ersten Raststruktur eingreifen, um die Rastverbindung zu bilden.

Das Vorschubelement kann in konkreten Ausführungsformen einen Bereich aufweisen, der einen kreuzförmigen Querschnitt mit vier senkrecht zueinander stehenden, sich in der Längsrichtung erstreckenden Stegen hat. Die erste Raststruktur ist dann vorzugsweise an mindestens einem von diesen Stegen ausgebildet.

In einer bevorzugten Ausführungsform weist das Einsatzelement einen Hauptabschnitt auf, an welchem, entlang der Längsrichtung beabstandet zueinander, drei Paare von in das Reservoir hineinragenden Armen ausgebildet sind, welche paarweise quer zur Längsrichtung einander gegenüberliegend angeordnet sind, wobei die Arme der in axialer Richtung distal und proximal angeordneten Paare jeweils ein sich quer zur axialen Richtung vom Einsatzelement nach aussen hin erstreckendes Hintergreifelement aufweisen, und wobei die Arme des dazwischen angeordneten Paares jeweils eine sich quer zur axialen Richtung und bezüglich des Einsatzelementes nach innen hin erstreckende Rastnase aufweisen.

Die Vorrichtung kann insbesondere auch als Mehrfachapplikator mit mehreren Reservoirs, z.B. als Doppelspritze oder Mehrfachspritze ausgebildet sein. In jedem der Reservoirs ist dann ein Kolben vorhanden, der mit einem Vorschubelement verbunden ist. Die Vorschubelemente sind dann an ihren proximalen Enden verbunden. In diesem Fall ist es ausreichend, wenn nur in der Seitenwand eines der Reservoire eine Fensteröffnung mit einem Einsatzelement der vorstehend beschriebenen Art vorhanden ist.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine perspektivische Explosionsansicht einer erfindungsgemässen Austragvorrichtung gemäss einer ersten Ausführungsform;
- Fig. 2: eine perspektivische Ansicht der in Fig. 1 gezeigten Austragvorrichtung in zusammengesetztem Zustand;
- Fig. 3: eine erste Seitenansicht der in Fig. 1 gezeigten Austragvorrichtung;
- Fig. 4: eine zweite Seitenansicht der in Fig. 1 gezeigten Austragvorrichtung von einer im Vergleich zur Fig. 3 senkrecht stehenden Blickrichtung;
- Fig. 5: eine Schnittansicht in der Ebene V-V der in Fig. 3 gezeigten Austragvorrichtung;
- Fig. 6: eine Teil-Schnittansicht in der Ebene VI-VI der in Fig. 5 gezeigten Austragvorrichtung mit bis zu einem proximalen Anschlag aus dem Gehäuse zurückgezogenem Kolben;
- Fig. 7: eine Teil-Schnittansicht in der Ebene VI-VI der in Fig. 5 gezeigten Austragvorrichtung mit bis zu einem distalen Anschlag in das Gehäuse vorgeschobenem Kolben;
- Fig. 8: eine erste Seitenansicht einer erfindungsgemässen Austragvorrichtung gemäss einer zweiten Ausführungsform;
- Fig. 9: eine zweite Seitenansicht der in Fig. 8 gezeigten Austragvorrichtung von einer im Vergleich zur Fig. 8 senkrecht stehenden Blickrichtung;
- Fig. 10: eine Seitenansicht einer erfindungsgemässen Austragvorrichtung gemäss einer dritten Ausführungsform; sowie
- Fig. 11: eine perspektivische Explosionsansicht einer erfindungsgemässen Austragvorrichtung gemäss einer vierten Ausführungsform.

### BESCHREIBUNG BEVORZUGTER AUSFÜHRUNGSFORMEN

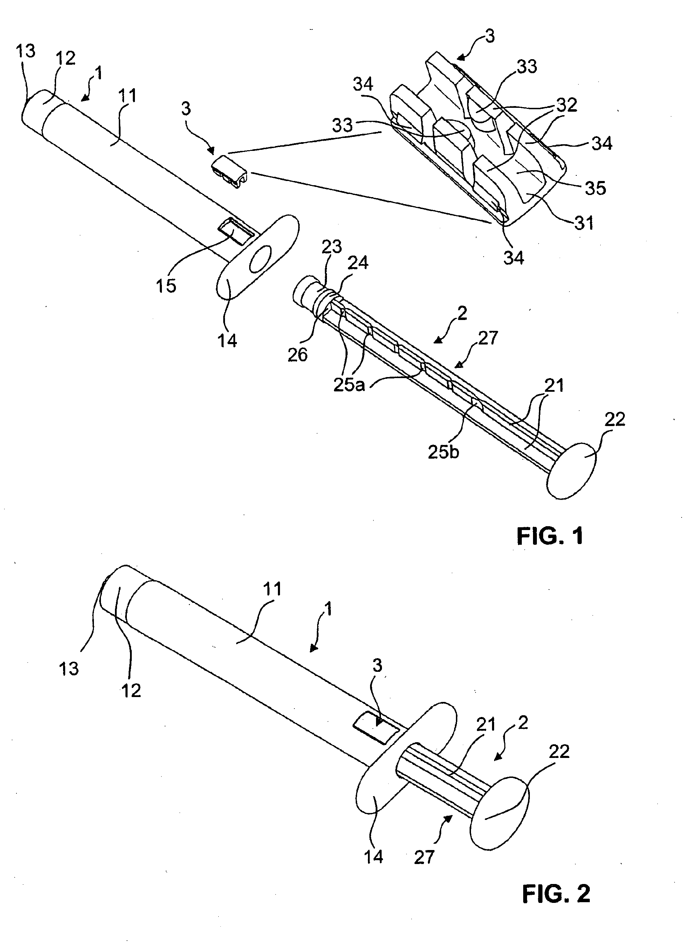

Die Figuren 1 bis 7 zeigen eine erfindungsgemässe Austragvorrichtung gemäss einer bevorzugten Ausführungsform. Bei der Austragvorrichtung dieser Ausführungsform handelt es sich um einen Spritze, welche beispielsweise für eine Verabreichung eines Medikaments oder zum Austragen eines Klebstoffs verwendet wird. Die Austragvorrichtung weist ein Gehäuse 1 auf, in welchem eine Kolbeneinheit 2 entlang einer axialen Richtung verschiebbar angeordnet ist.

Das Gehäuse 1, welches im Folgenden auch als Spritzenkörper bezeichnet wird, wird grösstenteils durch eine umlaufende, einen Hohlzylinder bildende Seitenwand 11 definiert, welche ein proximales und ein distales Ende aufweist. Die Seitenwand 11 begrenzt dabei ein sich in ihrem Innern erstreckendes, zylindrisches Reservoir 16. Durch seine zylindrische Form definiert das Reservoir 16 dabei eine Längsrichtung (axiale Richtung) und eine dazu radiale Richtung der Austragvorrichtung sowie eine Reservoirmittelachse. Anschliessend an das distale Ende der Seitenwand 11 ist ein im Wesentlichen zylindrisches Kopfteil 12 angeordnet, welches eine durchgehende, axiale Öffnung aufweist, die in eine distale, zentral im Kopfteil angeordnete Austrittsöffnung 13 mündet. Die Austrittsöffnung 13 ragt dabei in distaler Richtung über das Kopfteil 12 nach aussen hin vor und kann einen Luerkegel oder eine andere Verbindungsstruktur aufweisen, damit beispielsweise eine Injektionsnadel oder ein Zubehörteil, zum Beispiel ein Sprühaufsatz, am Gehäuse 1 angebracht werden kann. Im Reservoir 16 ist ein fluides Produkt aufnehmbar, bei dem es sich zum Beispiel um ein Medikament, einen Klebstoff oder um ein anderes Fluid handeln kann.

Am proximalen Ende des Gehäuses 1 sind auf zwei diametral gegenüberliegenden Seiten der Seitenwand 11 zwei radial nach aussen hin abstehende Halteflügel 14 angeordnet. Diese Halteflügel 14 erleichtern die Handhabung der Austragvorrichtung für den Benutzer, indem beispielsweise bei einem Vorschieben der Kolbeneinheit 2 in das Gehäuse 1 hinein jeweils ein Finger auf der distalen Seite von je einem Halteflügel 14 platziert werden kann, während die Kolbeneinheit 2 mittels Daumendruck vorgeschoben wird.

Im Bereich des proximalen Gehäuseendes ist innerhalb der Seitenwand 11 auf einer in eine senkrecht zu den Halteflügeln 14 weisenden Seite eine seitliche Fensteröffnung 15 vorhanden, welche sich hier in Umfangsrichtung der Seitenwand 11 über einen Winkelbereich von ca. 45° hin erstreckt. Die Fensteröffnung 15 hat eine rechteckige Form, wobei die längeren Rechtecksseiten jeweils entlang der Längsrichtung verlaufen. Die Fensteröffnung 15 ist in dieser Ausführungsform vollständig von der Seitenwand 11 umrandet. Dabei wird durch die Fensteröffnung 15 insbesondere das Reservoir 16 von einer radialen Richtung her zugänglich gemacht.

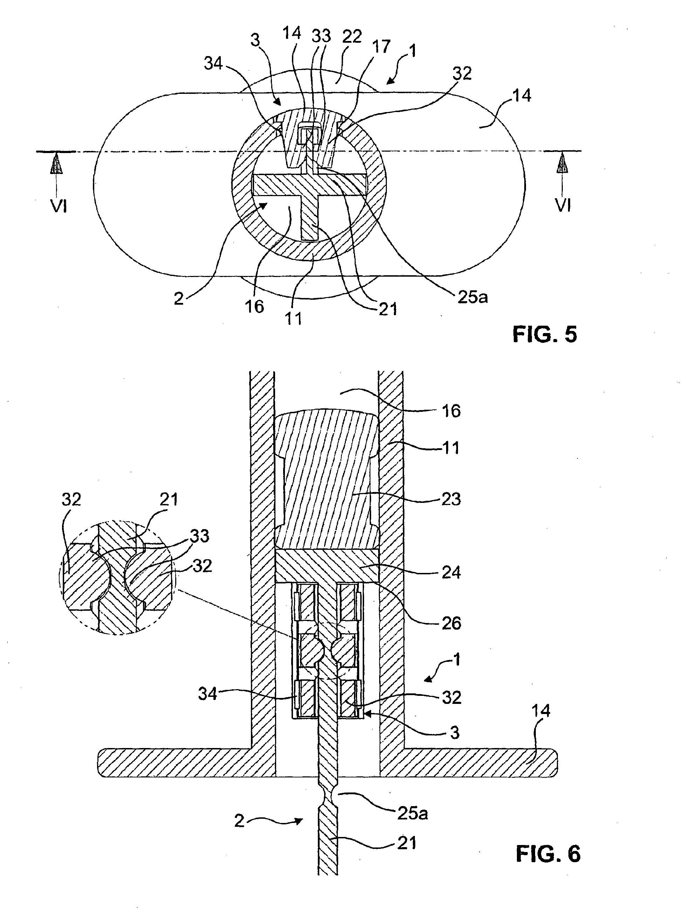

Im Randbereich der Öffnung 15 kann die Seitenwand 11 Flansche 17 (Fig. 5) aufweisen, welche sich jeweils entlang der beiden Längsseiten der rechteckigen Öffnung 15 in die Längsrichtung erstrecken. Diese Flansche 17 sind in radialer Richtung dünner ausgebildet als die Seitenwand 11 und bezüglich der radialen Richtung in etwa in der Mitte der Seitenwand 11 angeordnet.

Die Kolbeneinheit 2, weist eine insgesamt langgezogene Form auf und umfasst in diesem Ausführungsbeispiel einen distal angeordneten Kolben 23, eine damit verbundene Endplatte 24, eine einstückig mit der Endplatte 24 ausgebildete Kolbenstange 27, die ein Vorschubelement für den Kolben bildet, sowie eine proximal angeordnete Druckplatte 22.

Die Kolbenstange 27 weist hier eine kreuzförmige Querschnittsfläche auf mit vier senkrecht zueinander stehenden Stegen 21, die sich entlang der Längsrichtung erstrecken und auch als Längsrippen bezeichnet werden können. Die Kolbenstange 27 erstreckt sich über einen Grossteil der axialen Länge der Kolbeneinheit 2.

Am proximalen Ende der Kolbenstange 27 ist die sich senkrecht zur axialen Richtung erstreckende, ebene Druckplatte 22 angeordnet, welche einen im Vergleich zur restlichen Kolbeneinheit 2 vergrösserten Aussendurchmesser hat. Die Druckplatte 22 dient insbesondere als Daumenauflage für den Benutzer beim Vorschieben der Kolbeneinheit.

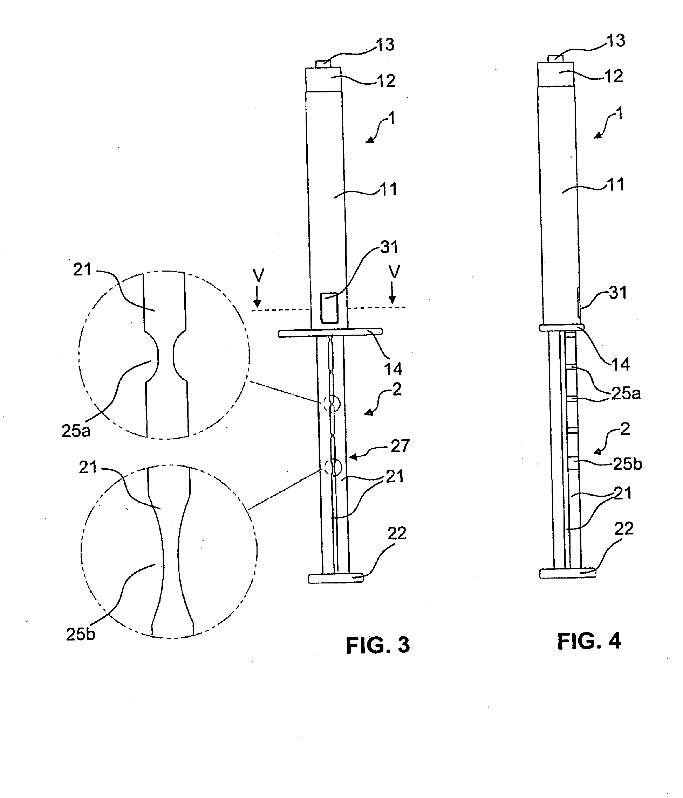

Wie beispielsweise in den Figuren 1 und 3 gezeigt ist, weist einer der Stege 21 in diesem Ausführungsbeispiel eine Mehrzahl von Rastkerben 25a und 25b auf. Die Rastkerben 25a, 25b sind in regelmässigen Abständen entlang der Längsrichtung angeordnet. Dabei stellen die Rastkerben 25a, 25b jeweils eine lokale Verjüngung des Stegs 21 dar, wobei die Verjüngungen bezüglich der beiden gegenüberliegenden Seiten des Stegs 21 symmetrisch ausgebildet sind. Die Rastkerben 25a, 25b erstrecken sich dabei jeweils in radialer Richtung durchgehend und gleichförmig über die ganze Längsrippe 21, was bedeutet, dass sich die Dicke der Längsrippe 21 in radialer Richtung an jeder Stelle und insbesondere auch im Bereich der Rastkerben 25a, 25b nicht verändert. In der Draufsicht auf den Steg 21 mit Rastkerben 25a, 25b von einer radialen Richtung her, sind die Rastkerben 25a, 25b, wie in Figur 3 ersichtlich, jeweils gerundet ausgebildet und stellen einander gegenüberliegende Ausnehmungen des Steges 21 in der Form von Kreissegmenten oder elliptischen Segmenten dar. Insbesondere weisen die Rastkerben 25a, 25b jeweils eine zur distalen Richtung geneigte (und gekrümmte) Schrägfläche sowie eine zur proximalen Richtung geneigte Schrägfläche auf. Die Rastkerben 25a bzw. 25b bilden jeweils eine Raststruktur.

Am distalen Ende der Kolbeneinheit 2 ist der Kolben 23 ausgebildet. Der Kolben 23 kann fest mit der Kolbenstange 27 verbunden sein, oder er kann ein separates, von der restlichen Kolbeneinheit 2 lösbares Teil darstellen. Im vorliegenden Beispiel ist der Kolben 23 mit einer Endplatte 24 verbunden. In distaler Richtung weist die Endplatte 24 eine Druckfläche auf, welche dazu ausgebildet ist, den Kolben 23 im Gehäuse 1 zu verschieben. In proximaler Richtung bildet die Endplatte 24 eine Anschlagfläche 26, die mit den Längsrippen 21 verbunden ist.

Die Kolbeneinheit 2 wird vom proximalen Gehäuseende her in das Reservoir 16 eingeschoben. Der Kolben 23 dichtet dabei das Reservoir 16 in die proximale Richtung fluiddicht ab. Dadurch ist er geeignet, das im Reservoir 16 aufgenommene fluide Produkt durch die Austrittsöffnung 13 zu pressen und dadurch aus der Austragvorrichtung auszutragen. Die Kolbeneinheit 2 ragt, wenn sie vollständig in das Gehäuse 1 vorgeschoben ist, am proximalen Gehäuseende aus dem Gehäuse 1 heraus. Die Stege 21 der Kolbenstange 27 erstrecken sich dabei in radialer Richtung fast bis zur Seitenwand 11 hin. Bei vollständig in das Gehäuse 1 vorgeschobener Kolbeneinheit 2 schlägt eine in distale Richtung gerichtete Endfläche des Kolbens 23 an einem in die proximale Richtung gerichteten Anschlag des Gehäuses 1 an. Vorzugsweise ist in einer solchen distalen Anschlagstellung des Kolbens 2 relativ zum Gehäuse 1 das im Reservoir 16 aufgenommene fluide Produkt im Wesentlichen vollständig aus dem Gehäuse 1 ausgestossen. Dieser Anschlag des Gehäuses 1 kann beispielsweise durch einen Übergangsbereich zwischen der Seitenwand 11 und dem Kopfteil 12 oder durch einen sich in radialer Richtung erstreckenden Gehäusebereich angrenzend zur Austrittsöffnung 13 gebildet sein.

Um zu verhindern, dass die Kolbeneinheit vollständig aus dem Gehäuse herausgezogen werden kann, und um das Austragen definierter Portionen zu erleichtern, ist in die Fensteröffnung 15 ein Einsatzteil 3 eingesetzt. Das Einsatzteil 3 weist in der vorliegenden Ausführungsform einen Hauptabschnitt 31 auf, der eine im Wesentlichen rechteckige Form hat und nach aussen hin entsprechend der Krümmung der Seitenwand 11 gekrümmt ist und bündig mit der Seitenwand abschliesst. Der Hauptabschnitt 31 weist insbesondere im Wesentlichen die Form und die Dimensionen der Öffnung 15 des Gehäuses 1 auf. Auf der radialen Innenseite des Hauptabschnitts 31 sind drei Paare von elastischen Armen 32 ausgebildet, die sich radial nach innen ins Reservoir 16 hinein erstrecken, wobei die beiden Arme 32 eines Paares jeweils quer zur Längsrichtung und zur radialen Richtung gegenüberliegend zueinander angeordnet sind. Die jeweiligen Arme 32 eines Paars definieren einen in Längsrichtung verlaufenden, gemeinsamen Kanal 35 des Einsatzteiles 3.

Das Einsatzteil 3 ist insgesamt dazu ausgebildet, einen Formschluss mit dem Gehäuse 1 zu bilden. Dazu weisen die am distalen und am proximalen Ende des Einsatzteiles 3 angeordneten Arme 32 jeweils auf ihrer äusseren, in Umfangsrichtung zum Rand der Öffnung 15 hin gerichteten Seite ein Hintergreifelement 34 auf. Die Hintergreifelemente 34 weisen insbesondere eine radial nach aussen hin gerichtete Haltefläche auf. Diese Halteflächen erstrecken sich jeweils parallel zur Seitenwand 11 in Umfangsrichtung. Auf ihrer der Haltefläche gegenüberliegenden Seite weisen die Hintergreifelemente 34 jeweils eine schräge Fläche auf, welche die Haltefläche mit den Ann 32 verbindet. Diese schrägen Flächen verlaufen jeweils abgewinkelt zur Seitenwand 11 und zur radialen Richtung.

Beim Einsetzen des Einsatzelementes 3 in die Öffnung 15 werden die beiden jeweils gegenüberliegend zueinander angeordneten Arme 32 leicht gegeneinander gedrückt, bis die Hintergreifelemente 34 über den Rand der Öffnung 15 hinweg bewegt sind, und das Einsatzelement 3 in der Öffnung 15 einschnappt. Die schrägen Flächen der Hintergreifelemente 34 erleichtern dabei das Einsetzen des Einsatzteiles 3 in die Öffnung 15 und ermöglichen ein Einschnappen der Hintergreifelemente 34 auf der Innenseite der Seitenwand 11. Die radial nach aussen weisenden Halteflächen der Hintergreifelemente 34 bilden dann einen Anschlag an der Innenseite der Seitenwand 11 an den Flanschen 17, wodurch das Einsatzteil 3 in der Öffnung 15 gehalten ist.

Das Einsetzen des Einsatzteiles 3 in die Öffnung 15 ist ausserdem dadurch erleichtert, dass die Aussenseiten der Arme 32 insgesamt jeweils leicht zum Kanal 35 hin geneigt sind. Zudem können nach aussen weisende Ecken der proximal und distal am Einsatzelement 3 angeordneten Armpaare, wie in Figur 1 gezeigt, abgerundet sein, um das Einsetzen des Einsatzteiles 3 in die Öffnung 15 weiter zu erleichtern.

Die Flansche 17 der Seitenwand 11 ermöglichen es, das Einsatzteil 3 derart auszugestalten, dass die Aussenseite des Hauptabschnitts 31 mit der Aussenseite der Seitenwand 11 bündig ist, wenn das Einsatzteil 3 in die Öffnung 15 eingesetzt ist (Figur 5). Nach Einschnappen des Einsatzteiles 3 in die Fensteröffnung 15 kann dadurch ein unbeabsichtigtes Entfernen des Einsatzteiles 3 durch den Benutzer verhindert werden. Ausserdem ermöglicht es der Flansch 17, dass die Hintergreifelemente 34 im eingesetzten Zustand des Einsatzteiles 3 nicht in das Reservoir 16 hineinragen.

Das bezüglich der Längsrichtung mittig angeordnete Paar von Armen 32 des Einsatzelementes 3 weist im Bereich der freien Enden der Arme zwei zueinander hin gerichtete Rastnasen 33 auf. In radialer Richtung sind die Rastnasen 33 im Vergleich zu den Hintergreifelementen 34 im in das Gehäuse 1 eingesetzten Zustand des Einsatzelementes 3 näher zur Reservoirmittelachse angeordnet. Sie weisen eine halbrunde Form auf, die komplementär zur Form der Rastkerben 25a der Kolbeneinheit 2 ist. Dabei ragen die Rastnasen 33 deutlich in den durch die Anne 32 gebildeten Kanal 35 des Einsatzteiles 3 hinein. Die zwei einander gegenüberliegenden Rastnasen 33 bilden zusammen mit den Armen 32, an denen sie jeweils ausgebildet sind, eine Raststruktur.

Wenn das Einsatzteil 3 in das Gehäuse 1 eingesetzt ist, ragen die Arme 32 in radialer Richtung durch die Öffnung 15 in das Reservoir 16 hinein. Durch dieses Hineinragen der Arme 32 bildet das Einsatzelement 3 für die Kolbeneinheit 2 einen proximalen Anschlag, der ein vollständiges Entfernen der Kolbeneinheit 2 vom Gehäuse 1 verhindert, wie in den Figuren 5 und insbesondere 6 gezeigt ist. Beim Herausziehen der Kolbeneinheit 2 aus dem Gehäuse 1 heraus schlägt die Endplatte 24 mit ihrer Anschlagfläche 26 an den distal am Einsatzelement 3 angeordneten Armen 32 an, wodurch die axiale Bewegung der Kolbeneinheit 2 relativ zum Gehäuse in proximaler Richtung wirksam begrenzt ist. In einer alternativen Ausführungsform könnte das Einsatzelement 3 durch das Hineinragen der Arme 32 in das Reservoir 16 zudem auch einen distalen Anschlag für die Kolbeneinheit 2 bilden, der ein axiales Vorschieben der Kolbeneinheit 2 in distaler Richtung begrenzt. Die Kolbeneinheit 2 würde dann im Bereich der Kolbenstange 27 ein entsprechendes, radial nach aussen ragendes Anschlagelement aufweisen, welches in einem gegenüber dem Einsatzelement 3 proximalen Bereich angeordnet wäre.

Im Zustand, in dem das Einsatzelement 2 in das Gehäuse 1 eingesetzt ist, verläuft der Steg 21 der Kolbeneinheit 2, welcher die Rastkerben 25a, 25b aufweist, durch den Kanal 35.

Dadurch rasten beim Verschieben der Kolbeneinheit 2 innerhalb des Gehäuses 1 die Rastnasen 33 des Einsatzelementes 3 nacheinander von den gegenüberliegenden Seiten des Steges 21 in die Rastkerben 25a, 25b ein. Um miteinander die Rastverbindung einzugehen, sind die Rastkerben 25a, 25b und die Rastnasen 33 relativ zueinander in einer Eingriffsrichtung bewegbar, die quer zur Längsrichtung und quer zur Richtung, in der das Einsatzelement 3 in die Fensteröffnung 15 eingesetzt ist, verläuft. Die Arme 32, welche die Rastnasen 33 aufweisen, sind dazu vorteilhaft leicht flexibel ausgebildet. Je nach Bewegung der Kolbeneinheit 2 in die distale oder in die proximale Richtung schlagen die Rastnasen 33 dabei an den in proximale Richtung bzw. in distale Richtung gerichteten Schrägflächen der Rastkerben 25a, 25b an. Das Vorschieben bzw. Zurückziehen der Kolbeneinheit 2 wird dadurch an diesen Rastpositionen der Kolbeneinheit 2 relativ zum Gehäuse 1 für den Benutzer spürbar erschwert. Dies kann beispielsweise dazu dienen, dem Benutzer das erfolgte Austragen oder Aufziehen einer bestimmten Dosis anzuzeigen. Ein weiteres Vorschieben bzw. Zurückziehen der Kolbeneinheit 2 ist bei Erreichen einer Rastposition erst nach Überwinden einer bestimmten Lösekraft wieder möglich.

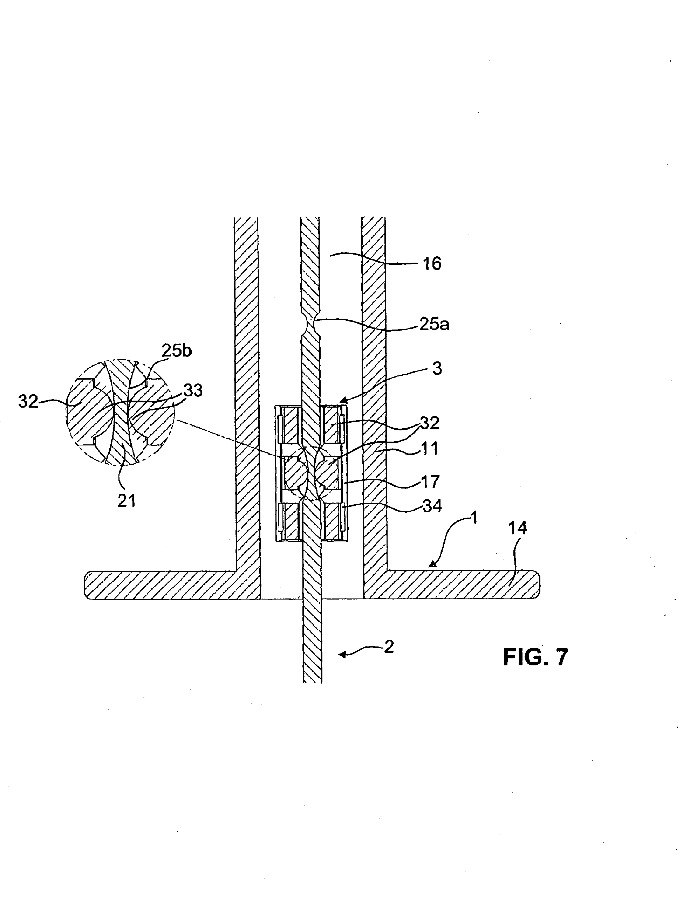

In der eingerasteten Stellung der Rastnase 33 in einer der Rastkerben 25a, 25b ist eine Bewegung der Kolbeneinheit 2 relativ zum Gehäuse 1 über einen gewissen axialen Bewegungsbereich ("Spiel") möglich. Die Rastkerben 25a sind derart ausgebildet, dass dieser axiale Bewegungsbereich minimal ist. Die am nächsten bei der Druckplatte 22 angeordnete Rastkerbe 25b hingegen weist im Vergleich zu den anderen Rastkerben 25a eine in axiale Richtung verlängerte Ausgestaltung auf (siehe Figur 3), wodurch der axiale Bewegungsbereich der Rastkerbe 25b in der Raststellung vergrössert ist (Figur 7). Somit ist in einer Stellung, in welcher die Rastnasen 33 in die Rastkerbe 25b einrasten, ein gewisses Spiel zwischen der Kolbeneinheit 2 und dem Gehäuse 1 in axialer Richtung möglich. Bevorzugt ist die verlängert ausgebildete Rastkerbe 25b derart an der Kolbenstange 27 angeordnet, dass sie dann mit der Rastnase 33 eine Rastverbindung eingeht, wenn sich die Kolbeneinheit 2 relativ zum Gehäuse 1 in einer distalen Anschlagstellung befindet. Die derart angeordnete und verlängert ausgebildete Rastkerbe 25b gewährleistet, dass die Kolbeneinheit 2 auch bei herstellungsbedingten Fertigungstoleranzen in der distalen Anschlagstellung eine Raststellung einnimmt.

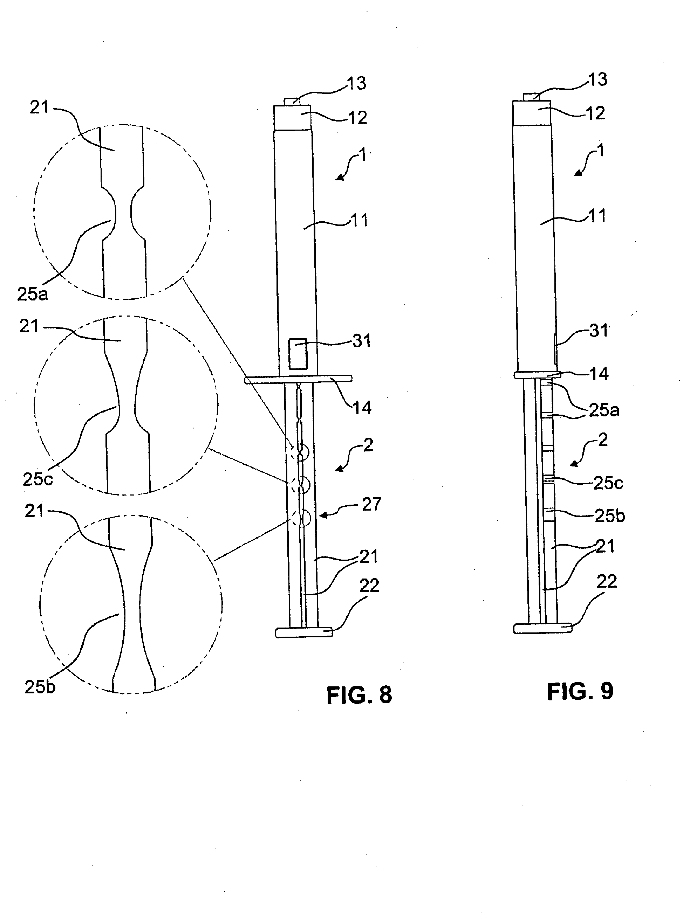

Eine zweite Ausführungsform der Austragvorrichtung, bei welcher die in proximaler Richtung im Vergleich zu allen Rastkerben 25a, 25b, 25c am zweitnäehsten zur Druckplatte 22 angeordnete Rastkerbe 25c eine besondere Form aufweist, ist in den Figuren 8 und 9 gezeigt. Die Rastkerbe 25c ist wie die Rastkerben 25a und 25b ebenfalls gerundet ausgebildet. Die Rastkerbe 25c ist jedoch in Bezug auf die axiale Richtung asymmetrisch ausgebildet, indem die Schrägfläche auf der distalen Seite der Rastkerbe 25c wesentlich flacher zur Reservoirmittelachse verläuft als auf der proximalen Seite. Beim Vorschieben der Kolbeneinheit 2 in das Gehäuse 1 hinein erfolgt das Einrasten dadurch spürbar langsamer und sanfter. Dies zeigt dem Benutzer beispielsweise das Erreichen von nur noch einer letzten verbliebenen Dosiseinheit in der Austragvorrichtung an. Beim Zurückziehen der Kolbeneinheit 2 aus dem Gehäuse 1 heraus ist für den Benutzer durch diese flacher ausgebildete distale Schrägfläche der Rastkerbe 25c eine geringere Lösekraft der Rastverbindung spürbar.

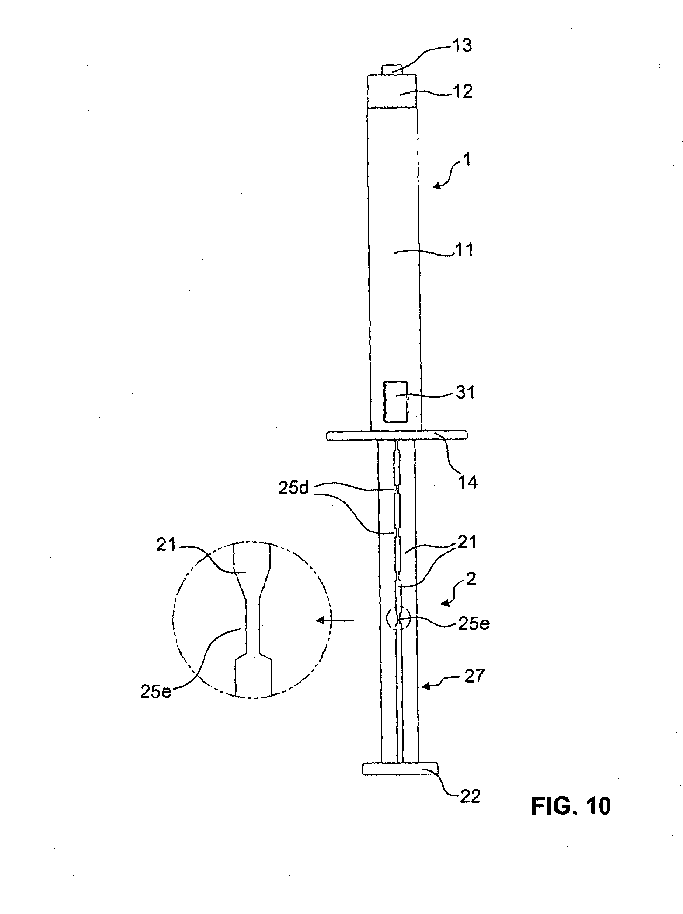

Eine weitere Ausführungsform ist in Figur 10 dargestellt, wo die Rastkerben 25d und 25e nicht gerundet, sondern eckig ausgebildet sind. Die eckige Ausgestaltung dieser Rastkerben 25d, 25e verändert das vom Benutzer gespürte Einrasten des Kolbens 2 im Einsatzelement 3 sowie die Lösekraft zum Lösen der Rastverbindung gegenüber der gerundeten Ausgestaltung der Rastkerben 25a, 25b. Die in proximaler Richtung zuhinterst angeordnete Rastkerbe 25e unterscheidet sich in dieser Ausführungsform von den anderen Rastkerben 25d durch ihre in Bezug auf die axiale Richtung asymmetrische Ausgestaltung. Diese asymmetrische Ausgestaltung der Rastkerbe 25e hat ein im Vergleich zu den anderen Rastkerben 25d abweichendes Rastverhalten zur Folge und zeigt dem Benutzer dadurch das vollständige Vorschieben der Kolbeneinheit 2 bzw. das erfolgte Verabreichen der letzten Dosis an. Ausserdem ist beim Vorschieben der Kolbeneinheit 2 die Lösekraft zum Lösen dieser Rastverbindung vermindert.

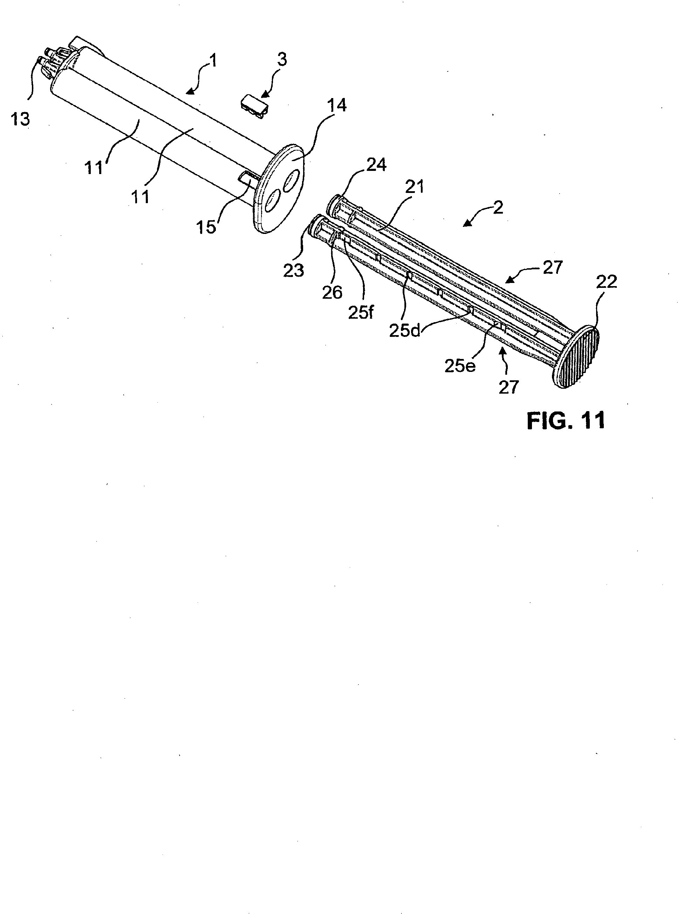

Figur 11 zeigt eine Ausführungsform, bei der die Austragvorrichtung als eine Doppelspritze ausgebildet ist. Dabei weist das Gehäuse 1 zwei parallele Reservoirs auf, die durch jeweils eine umlaufende Seitenwand 11 definiert sind und zur Aufnahme von jeweils einem Fluid dienen. Jedes der Reservoirs mündet in jeweils eine Austrittsöffnung 13, an die beispielsweise ein in der Zeichnung nicht dargestelltes, gemeinsames Mischelement angebracht werden kann, um die auszutragenden Substanzen der beiden Reservoirs miteinander zu vermischen. Die Kolbeneinheit 2 ist als ein Doppelkolben ausgebildet, bei dem die beiden langgezogenen Kolbenstangen 27 über die Druckplatte 22 miteinander verbunden sind. Rastkerben 25d, 25e und 25f sind in dieser Ausführungsform nur an einer der beiden Kolbenstangen 27 ausgebildet. Entsprechend ist auch nur in einer der Seitenwände 11 eine Öffnung 15 vorhanden, in welche ein Einsatzelement 3 einsetzbar ist. Die Rastkerben 25d, 25e und 25f weisen alle eine eckige Form auf, wobei die in axialer Richtung zuvorderst und zuhinterst angeordneten Rastkerben 25f und 25e jeweils im Vergleich zu den anderen Rastkerben 25d verlängert ausgebildet sind. Die am nächsten bei der Druckplatte 22 angeordnete Rastkerbe 25e ist zudem asymmetrisch ausgestaltet.

Das Gehäuse 1, die Kolbeneinheit 2 und das Einsatzelement 3 sind in den beschriebenen Ausführungsformen jeweils als Ganzes einstückig ausgebildet und aus Kunststoff in einem Spritzgussverfahren hergestellt. Dabei kann insbesondere die Kolbeneinheit 2 in einem Zweikomponenten-Spritzgussverfahren hergestellt sein und einen im Vergleich zur Kolbenstange 27 und zur Druckplatte 22 weicher ausgebildeten Kolben 23 aufweisen. Die Kolbeneinheit 2 kann aber auch zwei- oder mehrteilig ausgebildet sein, wobei dann insbesondere der Kolben 23 ein separates Teil darstellen kann. Das Einsatzelement 3 besteht vorteilhaft aus einem Material, das den Anforderungen angepasst ist. Es ist also zum Beispiel weniger spröde ausgebildet als das Gehäuse 1.

Die Erfindung ist selbstverständlich nicht auf die vorstehenden Ausführungsbeispiele beschränkt, und eine Vielzahl von Abwandlungen ist möglich. So kann das Einsatzelement auch anders als hier beschrieben ausgestaltet sein und beispielsweise anstatt durch Hintergreifelemente, welche radial von innen her das Einsatzelement in der Seitenwand 11 halten, mittels Halteklammern von aussen her am Gehäuse 1 gehalten sein. Es kann auch eine Karpule vorgesehen sein, in welcher das fluide Produkt gelagert ist. Die Karpule wäre dann in das Gehäuse eingesetzt und würde in ihrem Innern einen proximalen Verschlusskolben aufweisen, welcher durch die Kolbeneinheit vorschiebbar wäre, um das Fluid auszutragen. In einer anderen Ausführungsform könnte das Fluid auch in einem komprimierbaren Beutel gelagert sein, der in das Gehäuse eingesetzt ist. Anstatt Rastnasen könnten am Einsatzelement ausserdem auch Rastkerben und an der Kolbeneinheit entsprechend anstatt Rastkerben Rastnasen ausgebildet sein. Eine Vielzahl weiterer Abwandlungen ist denkbar.

**BEZUGSZEICHENLISTE**

| | | | |
|---|---|---|---|
| 1 | Gehäuse | 25c | Gerundete, asymmetrische |
| 11 | Seitenwand | | Rastkerbe |
| 12 | Kopfteil | 25d | Eckige Rastkerbe |
| 13 | Austrittsöffnung | 25e | Eckige, asymmetrische |
| 14 | Halteflügel | | Rastkerbe |
| 15 | Seitliche Öffnung | 25f. | Eckige, verlängerte Rastkerbe |
| 16 | Reservoir | 26 | Anschlagfläche |
| 17 | Flansch | 27 | Kolbenstange |
| 2 | Kolbeneinheit | 3 | Eins atzteil |
| 21 | Längsrippe | 31 | Hauptabschnitt |
| 22 | Druckplatte | 32 | Arm |
| 23 | Kolben | 33 | Rastnase |
| 24 | Endplatte | 34 | Hintergreifelement |
| 25a | Gerundete Rastkerbe | 35 | Kanal |
| 25b | Gerundete, verlängerte Rastkerbe | | |

## Patentansprüche

1. Austragvorrichtung zum Austragen eines fluiden Produktes, aufweisend
ein Gehäuse (1), welches ein Reservoir (16) für das fluide Produkt begrenzt, mit einer umlaufenden Seitenwand (11), einem offenen proximalen Gehäuseende und einem distalen Gehäuseende mit einer Austrittsöffnung (13),
einen Kolben (23), welcher im Gehäuse (1) entlang einer Längsrichtung verschiebbar angeordnet ist, um das fluide Produkt durch die Austrittsöffnung (13) hindurch aus dem Reservoir (16) auszustossen, und
ein mit dem Kolben (23) verbundenes Vorschubelement (27),
wobei in der Seitenwand (11) des Gehäuses (1) eine Fensteröffnung (15) ausgebildet ist,
wobei die Austragvorrichtung ein Einsatzelement (3) aufweist, welches quer zur Längsrichtung in die Fensteröffnung (15) eingesetzt ist und dabei ins Innere des Gehäuses (1) hineinragt,
und wobei das Vorschubelement (27) zumindest eine erste Raststruktur (25a; 25b; 25c; 25d; 25e) aufweist, und das Einsatzelement (3) eine zweite Raststruktur (32, 33) aufweist, und wobei die erste und zweite Raststruktur dazu ausgebildet sind, in mindestens einer Stellung des Vorschubelements (27) eine lösbare Rastverbindung einzugehen, um eine Verschiebung des Vorschubelements (27) in der Längsrichtung relativ zum Gehäuse (1) zu erschweren,
**dadurch gekennzeichnet, dass**
das Vorschubelement (27) am proximalen Gehäuseende aus dem Gehäuse (1) herausragt, und die erste Raststruktur (25a; 25b; 25c; 25d; 25e) und die zweite Raststruktur (32, 33) relativ zueinander in einer Eingriffsrichtung bewegbar sind, die quer zur Längsrichtung und quer zu einer Richtung, in der das Einsatzelement (3) in die Fensteröffnung (15) eingesetzt ist, verläuft, um miteinander die Rastverbindung einzugehen.

2. Austragvorrichtung gemäss Anspruch 1, wobei das Einsatzelement (3) mindestens ein Hintergreifelement (34) aufweist, um das Einsatzelement (3) in die Fensteröffnung (15) einzuschnappen.

3. Austragvorrichtung gemäss Anspruch 1 oder 2, wobei das Einsatzelement (3) nach aussen hin bündig mit dem Gehäuse (1) ausgebildet ist.

4. Austragvorrichtung gemäss einem der vorhergehenden Ansprüche, wobei das Einsatzelement (3) einen Anschlag für den Kolben (23) bei einer Bewegung des Kolbens (23) in Richtung der proximalen Gehäuseendes bildet.

5. Austragvorrichtung gemäss einem der vorhergehenden Ansprüche, wobei die erste Raststruktur (25a; 25b; 25c; 25d; 25e) und die zweite Raststruktur (32, 33) derart ausgebildet sind, dass die Rastverbindung durch Anwendung einer axialen Lösekraft entlang der Längsrichtung lösbar ist.

6. Austragvorrichtung gemäss Anspruch 5, wobei die erste Raststruktur (25a; 25b; 25c; 25d; 25e) und die zweite Raststruktur (32, 33) derart ausgebildet sind, dass die Rastverbindung durch Anwendung einer axialen Lösekraft entlang der Längsrichtung sowohl in proximaler als auch in distaler Richtung lösbar ist, wobei die axiale Lösekraft in distaler Richtung unterschiedlich von der axialen Lösekraft in proximaler Richtung ist.

7. Austragvorrichtung gemäss einem der vorhergehenden Ansprüche, wobei das Vorschubelement (27) eine Mehrzahl von entlang der Längsrichtung beabstandet zueinander angeordneten ersten Raststrukturen (25a; 25b; 25c; 25d; 25e) aufweist, die dazu ausgebildet sind, mit der zweiten Raststruktur (32, 33) in unterschiedlichen Positionen des Vorschubelements (27) eine lösbare Rastverbindung einzugehen.

8. Austragvorrichtung gemäss Anspruch 7, wobei zumindest zwei der ersten Raststrukturen (25a; 25b; 25c; 25d; 25e) derart unterschiedlich zueinander ausgestaltet sind, dass eine entlang der Längsrichtung wirkende axiale Lösekraft, die zum Lösen der Rastverbindung der jeweiligen ersten Raststruktur (25a; 25b; 25c; 25d; 25e) mit der zweiten Raststruktur (32, 33) erforderlich ist, jeweils unterschiedlich ist.

9. Austragvorrichtung gemäss Anspruch 7 oder 8,
wobei die ersten Raststrukturen (25a; 25b; 25c; 25d; 25e) und die zweite Raststruktur (32, 33) so ausgebildet sind, dass sie für mindestens eine Rastverbindung eine Bewegung des Vorschubelementes (27) relativ zum Gehäuse (1) über einen axialen Bewegungsbereich erlauben, ohne die Rastverbindung zu lösen,
wobei der Kolben (23) in eine distale Anschlagstellung verschiebbar ist, in der der Kolben (23) in distaler Richtung am Gehäuse (1) anschlägt, so dass eine weitere Bewegung des Kolbens (23) und des Vorschubelementes (27) relativ zum Gehäuse (1) in distaler Richtung verhindert wird, und
wobei eine ausgewählte erste Raststruktur (25b; 25e) derart ausgebildet ist, dass sie eine Rastverbindung mit der zweiten Raststruktur (32, 33) eingeht, wenn sich der Kolben (23) im Bereich seiner distalen Anschlagstellung befindet, und
wobei der axiale Bewegungsbereich des Vorschubelements (27) für diese Rastverbindung grösser ist als für mindestens eine andere Rastverbindung einer der restlichen ersten Raststrukturen (25a, 25c; 25d) mit der zweiten Raststruktur (32, 33).

10. Austragvorrichtung gemäss einem vorhergehenden Ansprüche, wobei die erste Raststrukfur zumindest eine Rastkerbe (25a; 25b; 25c; 25d; 25e) und die zweite Raststruktur (32, 33) zumindest eine Rastnase (33) aufweist.

11. Austragvorrichtung gemäss einem der vorhergehenden Ansprüche, wobei die zweite Raststruktur (32, 33) zwei flexibel ausgebildete Arme (32) umfasst, die quer zur Längsrichtung einander gegenüberliegend angeordnet sind und jeweils eine zum anderen Arm hin gerichtete Rastnase (33) aufweisen, und wobei die erste Raststruktur (25a; 25b; 25c; 25d; 25e) derart ausgebildet ist, dass diese zwei Rastnasen (33) von zwei gegenüberliegenden Seiten her in der ersten Raststruktur (25a; 25b; 25c; 25d; 25e) eingreifen, um die Rastverbindung zu bilden.

12. Austragvorrichtung gemäss einem der vorhergehenden Ansprüche, wobei das Einsatzelement (3) einen Hauptabschnitt (31) aufweist, an welchem, entlang der Längsrichtung beabstandet zueinander, drei Paare von in das Reservoir (16) hineinragenden Armen (32) ausgebildet sind, wobei die Arme (32) jedes Paares quer zur Längsrichtung einander gegenüberliegend angeordnet sind, wobei die Arme (32) der in Längsrichtung distal und proximal angeordneten Paare jeweils ein sich quer zur Längsrichtung vom Einsatzelement (3) nach aussen hin erstreckendes Hintergreifelement (34) aufweisen, und wobei die Arme (32) des dazwischen angeordneten Paares jeweils eine sich quer zur Längsrichtung und bezüglich des Einsatzelementes (3) nach innen hin erstreckende Rastnase (33) aufweisen.

13. Austragvorrichtung nach einem der vorhergehenden Ansprüche,
wobei das Gehäuse (1) ein erstes Reservoir (16) für ein erstes fluides Produkt und ein zweites Reservoir (16) für ein zweites fluides Produkt begrenzt,
wobei im ersten Reservoir (16) ein erster Kolben (23) verschiebbar angeordnet ist, um das erste fluide Produkt durch eine erste Austrittsöffnung (13) hindurch aus dem ersten Reservoir (16) auszustossen, und wobei im zweiten Reservoir (16) ein zweiter Kolben (23) verschiebbar angeordnet ist, um das zweite fluide Produkt durch eine erste Austrittsöffnung (13) hindurch aus dem ersten Reservoir (16) auszustossen,
wobei der erste Kolben (23) mit einem ersten Vorschubelement (27) verbunden ist, das am proximalen Gehäuseende aus dem Gehäuse (1) herausragt, wobei der zweite Kolben (23) mit einem zweiten Vorschubelement (27) verbunden ist, das am proximalen Gehäuseende aus dem Gehäuse (1) herausragt, und wobei das erste und das zweite Vorschubelement (27) proximal miteinander verbunden sind,
wobei die Fensteröffnung (15) in der Seitenwand (11) des Gehäuses (1) zum ersten Reservoir (16) hin ausgebildet ist, und
wobei das Einsatzelement (3) quer zur Längsrichtung in die Fensteröffnung (15) eingesetzt ist und dabei in das erste Reservoir (16) hineinragt.

## Claims

1. A discharge device for discharging a fluid product, comprising
a housing (1), which delimits a reservoir (16) for the fluid product, with a circumferential sidewall (11), an open proximal housing end, and a distal housing end with an outlet opening (13),
a piston (23), which is slidably arranged in the housing (1) along a longitudinal direction in order to discharge the fluid product from the reservoir (16) through the outlet opening (13),
and
an advancing element (27), connected to the piston (23),
wherein a window opening (15) is formed in the sidewall (11) of the housing (1),
wherein the discharge device comprises an insert element (3) that has been inserted into the window opening (15) transversely to the longitudinal direction, thus protruding into the interior of the housing (1),
and wherein the advancing element (27) comprises at least one first locking structure (25a; 25b; 25c; 25d; 25e), and the insert element (3) comprises a second locking structure (32, 33), and wherein the first and the second locking structures are designed in at least one position of the advancing element (27) to enter a detachable locking connection in order to render difficult any sliding of the advancing element (27) in longitudinal direction relative to the housing (1),
**characterized in that**
the advancing element (27) protrudes at the proximal housing end from the housing (1), and the first locking structure (25a; 25b; 25c; 25d; 25e) and the second locking structure (32, 33) are movable relative to each other in a direction of engagement that extends transversely to the longitudinal direction and transversely to a direction in which the insert element (3) has been inserted into the window opening (15) in order to, together, establish the locking connection.

2. The discharge device according to claim 1, wherein the insert element (3) comprises at least one engaging element (34) in order to snap the insert element (3) into the window opening (15).

3. The discharge device according to claim 1 or 2, wherein the insert element (3) is designed so as to be flush with the housing (1) towards the outside.

4. The discharge device according to any one of the preceding claims, wherein the insert element (3) forms an end stop for the piston (23) during movement of the piston (23) in the direction of the proximal housing end.

5. The discharge device according to any one of the preceding claims, wherein the first locking structure (25a; 25b; 25c; 25d; 25e) and the second locking structure (32, 33) are designed such that the locking connection can be detached with the application of an axial detachment force along the longitudinal direction.

6. The discharge device according to claim 5, wherein the first locking structure (25a; 25b; 25c; 25d; 25e) and the second locking structure (32, 33) are designed such that the locking connection can be detached both in the proximal and in the distal directions with the application of an axial detachment force along the longitudinal direction, wherein the axial detachment force in the distal direction differs from the axial detachment force in the proximal direction.

7. The discharge device according to any one of the preceding claims, wherein the advancing element (27) comprises a plurality of first locking structures (25a; 25b; 25c; 25d; 25e) arranged so as to be spaced apart from each other in longitudinal direction, with said first locking structures (25a; 25b; 25c; 25d; 25e) being designed in different positions of the advancing element (27) to enter a detachable locking connection with the second locking structure (32, 33).

8. The discharge device according to claim 7, wherein at least two of the first locking structures (25a; 25b; 25c; 25d; 25e) are designed differently relative to each other such that an axial detachment force acting along the longitudinal direction, which detachment force is required for detaching the locking connection of the respective first locking structure (25a; 25b; 25c; 25d; 25e) from the second locking structure (32, 33), is different in each case.

9. The discharge device according to claim 7 or 8,
wherein the first locking structures (25a; 25b; 25c; 25d; 25e) and the second locking structure (32, 33) are designed so that for at least one locking connection they permit movement of the advancing element (27) relative to the housing (1) over a determined axial movement region without the locking connection being disconnected as a result of this,
wherein the piston (23) is slidable to a distal end stop position in which the piston (23) in the distal direction comes to rest against the housing (1) so that further movement of the piston (23) and of the advancing element (27) relative to the housing (1) in the distal direction is prevented, and
wherein a selected first locking structure (25b; 25e) is designed in such a manner that it enters a locking connection with the second locking structure (32, 33) when the piston (23) is located in the region of its distal end stop position, and
wherein the axial movement region of the advancing element (27) for this locking connection is greater than it is for at least one other locking connection of one of the remaining first locking structures (25a; 25c; 25d) to the second locking structure (32, 33).

10. The discharge device according to any one of the preceding claims, wherein the first locking structure comprises at least one locking notch (25a; 25b; 25c; 25d; 25e), and the second locking structure (32, 33) comprises at least one locking catch (33).

11. The discharge device according to any one of the preceding claims, wherein the second locking structure (32, 33) comprises two flexibly designed arms (32) that are arranged transversely to the longitudinal direction so as to be opposite each other and in each case comprise a locking catch (33) pointing towards the other arm, and wherein the first locking structure (25a; 25b; 25c; 25d; 25e) is designed in such a manner that the two locking catches (33) engage the first locking structure (25a; 25b; 25c; 25d; 25e) from two opposite sides in order to form the locking connection.

12. The discharge device according to any one of the preceding claims, wherein the insert element (3) comprises a main section (31) on which, spaced apart from each other along the longitudinal direction, three pairs of arms (32) are formed that protrude into the reservoir (16), wherein the arms (32) of each pair are arranged transversely to the longitudinal direction opposite each other, wherein the arms (32) of the pairs that are arranged distally and proximally in longitudinal direction in each case comprise an engaging element (34) that extends transversely to the longitudinal direction from the insert element (3) towards the outside, and wherein the arms (32) of the pair arranged in between in each case comprise a locking catch (33) that extends transversely to the longitudinal direction and towards the inside relative to the insert element (3).

13. The discharge device according to any one of the preceding claims, wherein the housing (1) delimits a first reservoir (16) for a first fluid product, and a second reservoir (16) for a second fluid product,
wherein in the first reservoir (16) a first piston (23) is slidably arranged in order to discharge the first fluid product through a first outlet opening (13) from the first reservoir (16), and wherein in the second reservoir (16) a second piston (23) is slidably arranged in order to discharge the second fluid product through a first outlet opening (13) from the first reservoir (16),
wherein the first piston (23) is connected to a first advancing element (27) that on the proximal housing end protrudes from the housing (1), wherein the second piston (23) is connected to a second advancing element (27) that on the proximal housing end protrudes from the housing (1), and wherein the first advancing element and the second advancing element (27) are proximally interconnected with each other,
wherein the window opening (15) is formed in the sidewall (11) of the housing (1) towards the first reservoir (16), and
wherein the insert element (3) is inserted into the window opening (15) transversely to the longitudinal direction, thus protruding into the first reservoir (16).

## Revendications

1. Dispositif de distribution destiné à distribuer un produit fluide, présentant
un boîtier (1) qui délimite un réservoir (16) pour le produit fluide, avec une paroi latérale périphérique (11), une extrémité de boîtier proximale ouverte et une extrémité de boîtier distale avec une ouverture de sortie (13),
un piston (23) qui est disposé de manière déplaçable dans le boîtier (1) le long d'une direction longitudinale, afin d'éjecter le produit fluide à travers l'ouverture de sortie (13) hors du réservoir (16), et
un élément d'avance (27) connecté au piston (23), une ouverture de fenêtre (15) étant réalisée dans la paroi latérale (11) du boîtier (1),
le dispositif de distribution présentant un élément d'insertion (3) qui est inséré transversalement à la direction longitudinale dans l'ouverture de fenêtre (15) et qui pénètre en l'occurrence à l'intérieur du boîtier (1),
et l'élément d'avance (27) présentant au moins une première structure d'encliquetage (25a ; 25b ; 25c ; 25d ; 25e), et l'élément d'insertion (3) présentant une deuxième structure d'encliquetage (32, 33), et la première et la deuxième structure d'encliquetage étant réalisées de manière à établir une liaison par encliquetage amovible dans au moins une position de l'élément d'avance (27), afin de rendre difficile un déplacement de l'élément d'avance (27) dans la direction longitudinale par rapport au boîtier (1), **caractérisé en ce que**
l'élément d'avance (27) ressort du boîtier (1) au niveau de l'extrémité de boîtier proximale, et la première structure d'encliquetage (25a ; 25b ; 25c ; 25d ; 25e) et la deuxième structure d'encliquetage (32, 33) peuvent être déplacées l'une par rapport à l'autre dans une direction d'engagement qui s'étend transversalement à la direction longitudinale et transversalement à une direction dans laquelle l'élément d'insertion (3) est inséré dans l'ouverture de fenêtre (15), afin d'établir ensemble la liaison par encliquetage.

2. Dispositif de distribution selon la revendication 1, dans lequel l'élément d'insertion (3) présente au moins un élément d'engagement par l'arrière (34) afin d'encliqueter l'élément d'insertion (3) dans l'ouverture de fenêtre (15).

3. Dispositif de distribution selon la revendication 1 ou 2, dans lequel l'élément d'insertion (3) est réalisé en affleurement vers l'extérieur avec le boîtier (1).

4. Dispositif de distribution selon l'une quelconque des revendications précédentes, dans lequel l'élément d'insertion (3) forme une butée pour le piston (23) dans le cas d'un déplacement du piston (23) dans la direction de l'extrémité de boîtier proximale.

5. Dispositif de distribution selon l'une quelconque des revendications précédentes, dans lequel la première structure d'encliquetage (25a ; 25b ; 25c ; 25d ; 25e) et la deuxième structure d'encliquetage (32, 33) sont réalisées de telle sorte que la liaison par encliquetage puisse être desserrée en appliquant une force de desserrage axiale le long de la direction longitudinale.

6. Dispositif de distribution selon la revendication 5, dans lequel la première structure d'encliquetage (25a ; 25b ; 25c ; 25d ; 25e) et la deuxième structure d'encliquetage (32, 33) sont réalisées de telle sorte que la liaison par encliquetage puisse être desserrée en appliquant une force de desserrage axiale le long de la direction longitudinale à la fois dans la direction proximale et dans la direction distale, la force de desserrage axiale dans la direction distale étant différente de la force de desserrage axiale dans la direction proximale.

7. Dispositif de distribution selon l'une quelconque des revendications précédentes, dans lequel l'élément d'avance (27) présente une pluralité de premières structures d'encliquetage (25a ; 25b ; 25c ; 25d ; 25e) disposées à distance les unes des autres le long de la direction longitudinale, lesquelles sont réalisées de manière à établir une liaison par encliquetage desserrable avec la deuxième structure d'encliquetage (32, 33) dans différentes positions de l'élément d'avance (27).

8. Dispositif de distribution selon la revendication 7, dans lequel au moins deux des premières structures d'encliquetage (25a ; 25b ; 25c ; 25d ; 25e) sont configurées de manière différente l'une de l'autre de telle sorte qu'une force de desserrage axiale agissant le long de la direction longitudinale, qui est nécessaire pour desserrer la liaison par encliquetage de la première structure d'encliquetage respective (25a ; 25b ; 25c ; 25d ; 25e) avec la deuxième structure d'encliquetage (32, 33), soit dans chaque cas différente.

9. Dispositif de distribution selon la revendication 7 ou 8, dans lequel
les premières structures d'encliquetage (25a ; 25b ; 25c ; 25d ; 25e) et la deuxième structure d'encliquetage (32, 33) sont réalisées de telle sorte qu'elles permettent, pour au moins une liaison par encliquetage, un déplacement de l'élément d'avance (27) par rapport au boîtier (1) sur une plage de déplacement axiale sans desserrer la liaison par encliquetage,
le piston (23) pouvant être déplacé dans une position de butée distale dans laquelle le piston (23) bute dans la direction distale contre le boîtier (1), de telle sorte qu'un déplacement supplémentaire du piston (23) et de l'élément d'avance (27) par rapport au boîtier (1) dans la direction distale soit empêché, et
une première structure d'encliquetage sélectionnée (25b ; 25e) étant réalisée de telle sorte qu'elle établisse une liaison par encliquetage avec la deuxième structure d'encliquetage (32, 33) lorsque le piston (23) se trouve dans la région de sa position de butée distale, et
la région de déplacement axiale de l'élément d'avance (27) pour cette liaison par encliquetage étant plus grande que pour au moins une autre liaison par encliquetage de l'une des premières structures d'encliquetage restantes (25a ; 25c ; 25d) avec la deuxième structure d'encliquetage (32, 33).

10. Dispositif de distribution selon l'une quelconque des revendications précédentes, dans lequel la première structure d'encliquetage présente au moins une encoche d'encliquetage (25a ; 25b ; 25c ; 25d ; 25e) et la deuxième structure d'encliquetage (32, 33) présente au moins un ergot d'encliquetage (33).

11. Dispositif de distribution selon l'une quelconque des revendications précédentes, dans lequel la deuxième structure d'encliquetage (32, 33) comprend deux bras (32) réalisés sous forme flexible qui sont disposés à l'opposé l'un de l'autre transversalement à la direction longitudinale et qui présentent chacun un ergot d'encliquetage (33) orienté vers l'autre bras, et dans lequel la première structure d'encliquetage (25a ; 25b ; 25c ; 25d ; 25e) est réalisée de telle sorte que ces deux ergots d'encliquetage (33) viennent en prise depuis deux côtés opposés dans la première structure d'encliquetage (25a ; 25b ; 25c ; 25d ; 25e), afin d'établir la liaison par encliquetage.

12. Dispositif de distribution selon l'une quelconque des revendications précédentes, dans lequel l'élément d'insertion (3) présente une portion principale (31) au niveau de laquelle sont réalisées trois paires de bras (32) pénétrant dans le réservoir (16) à distance les unes des autres le long de la direction longitudinale, les bras (32) de chaque paire étant disposés en regard l'un de l'autre transversalement à la direction longitudinale, les bras (32) des paires disposées distalement et proximalement dans la direction longitudinale présentant chacun un élément d'engagement par l'arrière (34) s'étendant transversalement à la direction longitudinale vers l'extérieur depuis l'élément d'insertion (3), et les bras (32) de la paire disposée entre elles présentant à chaque fois un ergot d'encliquetage (33) s'étendant transversalement à la direction longitudinale et vers l'intérieur par rapport à l'élément d'insertion (3).

13. Dispositif de distribution selon l'une quelconque des revendications précédentes, dans lequel le boîtier (1) délimite un premier réservoir (16) pour un premier produit fluide et un deuxième réservoir (16) pour un deuxième produit fluide,
un premier piston (23) étant disposé de manière déplaçable dans le premier réservoir (16), afin d'éjecter le premier produit fluide à travers une première ouverture de sortie (13) hors du premier réservoir (16), et un deuxième piston (23) étant disposé de manière déplaçable dans le deuxième réservoir (16) afin d'éjecter le deuxième produit fluide à travers une première ouverture de sortie (13) hors du premier réservoir (16),
le premier piston (23) étant connecté à un premier élément d'avance (27) qui fait saillie hors du boîtier (1) au niveau de l'extrémité de boîtier proximale, le deuxième piston (23) étant connecté à un deuxième élément d'avance (27) qui fait saillie hors du boîtier (1) au niveau de l'extrémité de boîtier proximale, et le premier et le deuxième élément d'avance (27) étant connectés proximalement l'un à l'autre,
l'ouverture de fenêtre (15) étant réalisée dans la paroi latérale (11) du boîtier (1) vers le premier réservoir (16), et
l'élément d'insertion (3) étant inséré transversalement à la direction longitudinale dans l'ouverture de fenêtre (15) et pénétrant en l'occurrence dans le premier réservoir (16).
